## Europäisches Patentamt

## European Patent Office

## Office européen des brevets

(19)

(11) Veröffentlichungsnummer: **0 052 213**
**B1**

(12) # EUROPÄISCHE PATENTSCHRIFT

(45) Veröffentlichungstag der Patentschrift:
**02.05.85**

(21) Anmeldenummer: **81107886.4**

(22) Anmeldetag: **03.10.81**

(51) Int. Cl.⁴: **C 08 J 11/00,** C 08 G 65/20,
C 08 G 65/30

(54) Verfahren zur Depolymerisation von Polytetramethylenglykolether.

(30) Priorität: **14.11.80 DE 3042960**

(43) Veröffentlichungstag der Anmeldung:
**26.05.82 Patentblatt 82/21**

(45) Bekanntmachung des Hinweises auf die Patenterteilung:
**02.05.85 Patentblatt 85/18**

(84) Benannte Vertragsstaaten:
**BE DE FR GB IT NL**

(56) Entgegenhaltungen:
**EP - A - 0 001 761**
**EP - A - 0 006 107**
**FR - A - 2 250 786**
**GB - A - 854 958**

(73) Patentinhaber: **BASF Aktiengesellschaft,
Carl-Bosch-Strasse 38, D-6700 Ludwigshafen (DE)**

(72) Erfinder: **Mueller, Herbert, Dr., Carostrasse 53,
D-6710 Frankenthal (DE)**
Erfinder: **Huchler, Otto Hermann, Dr.,
Weinbietstrasse 38, D-6703 Limburgerhof (DE)**

ACTORUM AG

## Beschreibung

Diese Erfindung betrifft ein Verfahren zur Depolymerisation von Polytetramethylenglykolether.

Polytetramethylenglykolether, die als hochwertige polymere Glykole zur Herstellung von Polyurethanharzen Verwendung finden, werden durch Polymerisation von Tetrahydrofuran gewonnen. Im allgemeinen werden Polymerisationsprodukte mit Molekulargewichten zwischen 500 und 3000 benötigt.

Werden die Apparaturen zur Herstellung des Polytetrahydrofurans von einer Molekulargewichtseinstellung auf eine andere umgestellt, so fallen durch Produktvermischung nicht typgerechte Fraktionen an, die sich aus Polymeren unterschiedlicher Molekulargewichtsbereiche zusammensetzen. Um diese nicht typgerechte Ware einer wirtschaftlich tragbaren Verwendung zuzuführen, ist es beispielweise erstrebenswert, sie zu Monomeren, nämlich in das Tetrahydrofuran, zurückzuverwandeln. Dieses Bedürfnis tritt auch immer dann ein, wenn durch unkontrollierte Vorgänge bei der Synthese Polymere anfallen, die der verlangten engen Spezifikation nicht entsprechen.

Bei der Herstellung von Polytetramethylenglykolether wird das Polymere, z.B. nach dem Verfahren der US-PS 2 751 419, durch wässrige extraktive Behandlung gereinigt. Die dabei erhältlichen Abwässer führen gelösten Polytetramethylenglykolether mit sich. Sie weisen deshalb einen sehr hohen chemischen Sauerstoffbedarf (COD) auf und belasten die Vorfluter. In der US-PS 4 115 408 wird ein Verfahren zur Depolymerisation von Polytetramethylenglykolether zu Tetrahydrofuran beschrieben, bei dem man solche Abwässer mit Schwefelsäure auf 150°C erhitzt. Dieses Verfahren ist vor allem aus zweierlei Gründen ungünstig. Erstens muss man bei Anwendung verhältnismässig hoch konzentrierter Säure bei 150°C mit erheblichen Korrosionsschwierigkeiten rechnen und zweitens ist nach durchgeführter Depolymerisation die verdünnte wässrige Schwefelsäure zu neutralisieren, ehe man sie in die Vorfluter ablassen kann, wodurch diese mit einer erheblichen Salzfracht belastet werden.

Es ist auch schon vorgeschlagen worden, Polytetrahydrofuran durch Pyrolyse abzubauen (Makromolekulare Chemie 81 (1965), Seiten 38 bis 50). Diese Methode weist aber den Nachteil auf, dass die Zersetzung uneinheitlich abläuft und eine Vielzahl von Pyrolyseprodukten gebildet werden.

Es war deshalb nach einem Verfahren zu suchen, das es erlaubt, Polytetramethylenglykolether auf einfachere Weise und unter Vermeidung einer Umweltbelastung durch Salzfracht oder Säure quantitativ in Tetrahydrofuran zurückzuspalten.

Diese Aufgabe wird dadurch gelöst, dass man die Depolymerisation von Polytetramethylenglykolether erfindungsgemäss durch Erhitzen von Polytetramethylenglykolether in Gegenwart von Bleicherden auf Temperaturen von 90 bis 180°C, vorzugsweise 100 bis 150°C vornimmt.

Durch das Verfahren der Erfindung wird das Polymere auf besonders vorteilhafte Weise und ohne Ausbeuteverlust in das Monomere zurückgespalten. Das erhaltene Monomere ist zudem von hervorragender Reinheit und kann deshalb nach Trocknung ohne weitere Behandlung mit sehr gutem Erfolg in die Polymerisation zurückgeführt werden.

Bleicherden, die sich zur Durchführung des erfindungsgemässen Verfahrens eignen, sind natürlich vorkommende wasserhaltige Aluminiumhydrosilikate aus der Montmorillonit-Gruppe, die bekanntlich vor der Anwendung meist durch eine Säurebehandlung gereinigt, aufgeschlossen und aktiviert werden.

Für die Depolymerisation der Polymethylenglykolether wird nur eine geringe Menge der Bleicherde benötigt. Vorteilhafte Ergebnisse werden erzielt, wenn man sie in einer Konzentration von z.B. 0,1 bis 5 Gew.%, bezogen auf das Polymere, anwendet. Natürlich können auch geringere oder grössere Mengen zur Anwendung kommen. Zweckmässigerweise wird man 1 bis 2 Gew.% an Bleicherde anwenden. Die einmal verwendete Bleicherde kann beliebig oft für eine erneute Depolymerisation verwendet werden.

Zur Durchführung des Depolymerisationsvorganges wird das Polymere mit der Bleicherde gemischt und auf Reaktionstemperatur erhitzt. Etwa ab 90 bis 100°C setzt die Reaktion ein. Sie ist bei 100 bis 130°C genügend schnell, um die Umsetzung technisch durchzuführen. Gibt man z.B. eine Bleicherdenmenge von 1 Gew.% zum Polymeren und hält die Reaktionstemperatur auf 130°C, so ist die Depolymerisation nach etwa 3 bis 4 Stunden vollständig abgelaufen. Im Zersetzungsgefäss befindet sich keine organische Substanz mehr, sondern lediglich der eingesetzte anorganische Katalysator. Er kann für eine erneute Behandlung direkt verwendet werden. Werden höhere Temperaturen oder höhere Konzentrationen an Bleicherde angewendet, so läuft die Reaktion schneller ab.

Wegen der Wiederverwendbarkeit des eingesetzten Katalysators ist die spezifische Katalysatoreinsatzzahl sehr gering. Will man den Katalysator dennoch entfernen, so kann er als inertes, natürliches anorganisches Material gefahrlos deponiert werden.

Dass man Polytetramethylenglykolether mit Bleicherden depolymerisieren kann, ist sehr überraschend, da aus der US-PS 3 433 829 bekannt ist, dass man durch Behandlung von Tetrahydrofuran mit Bleicherden bei Temperaturen von 20 bis 200°C Polytetrahydrofuran erhält.

Die in dem Beispiel genannten Teile sind Gewichtsteile.

## Beispiel

Ein Reaktionsgefäss, das mit Heizung, Rührer und absteigendem Kühler ausgerüstet ist, wird mit 400 Teilen Polytetramethylenglykolether vom Molekulargewicht 2000 und 4 Teilen TONSIL® OP-

TIMUM FF beschickt und anschliessend unter Rühren auf 130°C erhitzt. TONSIL® OPTIMUM FF ist eine im Handel erhältliche Bleicherde, der Firma Süd Chemie AG, München, mit dem Schüttgewicht 450 g/l.

Oberhalb von 100°C beginnt die Tetrahydrofuran-Abspaltung. Nach 3 Stunden werden 399 bis 400 Teile Tetrahydrofuran in der dem Kühler angeschlossenen Vorlage erhalten. Als Rückstand bleiben im Rührbehälter 4 Teile der eingesetzten pulverigen Bleicherde. Im erhaltenen Tetrahydrofuran werden neben geringen Wassermengen, die aus der eingesetzten Bleicherde und der Depolymerisation stammen, keine Verunreinigungen durch gaschromatographische Analyse festgestellt.

Mit der im Rührbehälter verbliebenen nun wasserfreien Bleicherde werden noch je 2mal 400 Teile Polytetramethylenglykolether vom Molekulargewicht 1000 und 650 wie oben beschrieben depolymerisiert. Eine Verringerung der Katalysatoraktivität wird hierbei nicht festgestellt. Auch diese Umsetzungen verlaufen quantitativ.

## Patentansprüche

1. Verfahren zur Depolymerisation von Polytetramethylenglykolether, dadurch gekennzeichnet, dass man Polytetramethylenglykolether in Gegenwart von Bleicherde auf Temperaturen von 90 bis 180°C erhitzt.

2. Verfahren nach Anspruch 1, dadurch gekennzeichnet, dass man auf Temperaturen von 100 bis 150°C erhitzt.

3. Verfahren nach Anspruch 1, dadurch gekennzeichnet, dass man, bezogen auf Polytetramethylenglykolether, 0,1 bis 5 Gewichtsprozent an Bleicherde anwendet.

## Claims

1. A process for depolymerizing polytetramethylene glycol ethers, wherein the polytetramethylene glycol ethers are heated in the presence of a bleaching earth at from 90 to 180°C.

2. A process as claimed in claim 1, wherein heating is effected at from 100 to 150°C.

3. A process as claimed in claim 1, wherein from 0.1 to 5 per cent by weight, based on polytetramethylene glycol ether, of bleaching earth is used.

## Revendications

1. Procédé pour dépolymériser du poly(éther de tétraméthylène-glycol), caractérisé en ce que l'on chauffe le poly(éther de tétraméthylène-glycol) en présence de terre décolorante entre 90 et 180°C.

2. Procédé suivant la revendication 1, caractérisé en ce que le chauffage est réalisé entre 100 et 150°C.

3. Procédé suivant la revendication 1, caractérisé en ce que l'on emploie entre 0,1 et 5% du poids du poly(éther de tétraméthylène-glycol) de terre décolorante.